# EUROPEAN PATENT APPLICATION

(11) **EP 4 166 005 A1**
(43) Date of publication of application: **19.04.2023**
(21) Application number: 20737509.8
(22) Date of filing: 12.06.2020
(51) Int. Cl.: A23L 13/00

(54) **METHOD FOR THE SYNTHESIS OF AN EDIBLE AND STERILIZABLE POROUS 3D SCAFFOLD SUITABLE FOR USE IN THE LARGE-SCALE PRODUCTION OF CULTURED MEAT**

(71) Applicant: Biotech Foods S.L., 20018 Donostia-San Sebastián (ES)
(72) Inventor: VILA, Mercedes, 20018 Donostia-San Sebastián (ES); CORTIZO, Diego, 20018 Donostia-San Sebastián (ES)
(74) Representative: Balder IP Law, S.L.
(86) International application number: PCT/ES2020/070385
(87) International publication number: WO 2021/250290

(57) **Abstract**

The present invention relates to a method for the large-scale synthesis of an edible and hot steam sterilizable macroporous three-dimensional (3D) scaffold which comprises biocompatible polymers with interconnected pores as a support material for adherent cell growth, proliferation and differentiation, which may be used to obtain tissue with nutritive content and/or cultured meat. These scaffolds are suitable for supporting cell tissue growth for biomedical or food applications.

## Description

### TECHNICAL FIELD

The present invention relates to a method for the large-scale synthesis of an edible and sterilizable macroporous three-dimensional (3D) scaffold which comprises biocompatible polymers, with interconnected pores as a support material for adherent cell growth, proliferation and differentiation, which may be used to of *in vitro* engineered tissues, also known in the art as cultured meat, cultivated meat, cell based meat, cellular meat and/or clean meat,. These scaffolds are suitable for supporting cell tissue proliferation for biomedical or food applications.

### STATE OF THE ART

Human population growth, which is expected to increase continuously (reaching 9,770 million by 2050 and 11,180 million at the turn of the century), will bring along greater waste production and the nutrient requirements of our species will continue to grow as we do. Meat figures prominently among protein foods.

In the last 40 years, global meat production has grown significantly, due mainly to the development of countries. This growth has limited the production system, adversely affecting the environment in terms of natural resources (land and water), human health (pandemics) and animal well-being (animal suffering), due to which the consumption of cultured meat significantly contributes to feeding all the inhabitants of the planet by helping to create a more sustainable system. The FAO estimates that in the next 40 years the demand for animal protein will continue to grow and, in turn, the challenges for satisfying that demand. The current production model will not be able to satisfy this demand if not combined with new strategies such as cultured meat or meat produced by *in vitro* cultivation of muscle cells previously extracted from an animal. Accordingly, there is a need for a solution to demands for alternative to meat produced from live animals.

Most of the research carried out on cultured meat can be considered variants or applications of tissue engineering, which is the discipline of biomedicine which, by combining cells, materials and engineering tools, attempts to design functional biological structures for repairing, replacing or regenerating damaged tissues. The progress made in this field is based mainly on the use of three-dimensional scaffolds/structures for tissue proliferation in a large variety of biomedical applications such as the regeneration of bone tissue, heart tissue, liver tissue, etc.

The patent application US 2006/0121006 A1, related to the use of cultured animal cells for the production of nutritional or therapeutic products wherein said cells are derived from rare or endangered species. Edible products containing meat, suitable for human and non-human consumption, whether as food or as food supplements, are also included. The invention also relates to the use of large-scale culture methods for the proliferation of the cells prior to being added to said products. The invention encompasses the methods for manufacturing the products, the products themselves and the methods of use of the products. Preference is shown for the culture of cells anchored to supports, particularly for supports in the form of microstructures (microspheres, fibres, etc.) of different materials, such as collagen, chitin, polystyrene, polyester or polypropylene, the choice of which will depend on the final use of the product and on whether the microstructure can remain in the final product or not.

Scaffolds based on natural polymers have demonstrated great potential for medical and pharmaceutical applications. The matrices formed by these biopolymers, some with the capabilities of hydrogel behavior, offer a series of advantages in terms of biocompatibility and biodegradability in comparison with the matrices formed from synthetic polymers. Thanks to their inherent properties, such as their great capacity to absorb water (swelling/hydration), biopolymer hydrogels are capable of efficiently encapsulating and releasing a large variety of hydrophobic and hydrophilic therapeutic molecules in a controlled manner, including nucleic acids, proteins, antibodies, etc. In biomedical applications, they have been used, for example, for cell encapsulation. The cells are generally mixed with the polymer solution prior to the gelation process (formation of the hydrogel). This method makes it possible to encapsulate individual cells or a mixture of various cell types in a certain volume of polymer material, both spherical and in the form of fibers. The main limitation of cell encapsulation is maintaining an appropriate diffusion balance for transporting oxygen and nutrients to the cells, due to which strategies that increase the porosity and permeability of the hydrogel have been developed, giving rise to porous hydrogels. The internal porous structure of these hydrogels enables cell adhesion and proliferation.

The production of macroporous 3D scaffolds that supports the proliferation of different types of cells to generate different sorts of tissues is crucial for a wide number of applications such as the regenerative medicine (bone tissue, connective tissue, muscular tissue...) or lately for the production of cultured meat, among others, although work has been mainly carried out at small scale and in basic research. Normally, regenerative medicine research it has been proposed that the polymeric matrices intended for tissue regeneration become slowly degraded as the new tissue is formed.

As mentioned previously, a developing field within biofabrication/biomanufacturing aims at producing animal products for human consumption (i.e., cultured meat) without killing animals. As such it shares the challenge of producing large quantities of cells and raises other challenges for scaffolds. Thus, it would be beneficial to develop a new type of scaffold with characteristics that may include: (1) the scaffold should be edible so they can be incorporated into the final comestible product (e.g., no synthetic material, no toxic chemical used for their formation); (2) the scaffolds need to have been formed of an animal-free composition to assure that the final product retains its no animal kill character (e.g., no collagen, gelatin, etc.); (3) production of the scaffolds needs to be scalable and low cost; and (4) the scaffolds may bring additional features to the final product (e.g., gustatory benefit, mouthful feeling, health benefits i.e., higher content of fibers, etc.).

One of the main challenges of supporting large scale cultured meat growth using the scaffolding technique is the compatibility of the scaffolds with the sterilization techniques used in large-scale bioreactors. The sterilization processes are based on a combination of steam, pressure and time and are operated at high temperature and pressure in order to kill microorganisms and spores. The scaffolds known in the art are not suitable to accomplish this process without losing its properties for tissue engineering.

Given the growing demand for substitutes for meat obtained directly from animals, it would be interesting to develop new methods for obtaining edible, sterilizable and 3D macroporous scaffolds, useful to obtain nutritive tissues also known as cultured meat, that overcoming the aforementioned limitations, mainly that the scaffold obtained maintain their properties, such as, mechanical strength, porosity and interconnectivity of the pores, when these scaffolds are subjected to the sterilization techniques used in large-scale culture bioreactors.

### DESCRIPTION OF THE INVENTION

The present invention describes a method for obtaining a sterilizable macroporous 3D scaffold which comprises at least a biocompatible polymer and also having interconnected pores, wherein the sterilizable macroporous 3D scaffold obtainable by the method disclosed herein, may be used as a support material for adherent cell growth, proliferation and differentiation, and may further be applied for the biofabrication of engineered tissues, also named or known for the skilled person as cultured meat, cultivated meat, cell based meat, cellular meat and/or clean meat, which are suitable for human consumption, and/or for tissue regeneration applications.

The method of the invention allows to obtain a sterilizable scaffold without losing its properties, such as, mechanical strength, porosity and interconnectivity of the pores, as scaffold for cell proliferation. The composition and structure of the scaffold obtained after the sterilization step are adequate for cell adhesion and the crosslinking and sterilization steps not only do not change the applicability of the materials due to possible degradation/denaturalization, but even improve the mechanical properties for supporting cell proliferation.

Sterilization method, preferably by hot steam, are commonly used in healthcare facilities and in cell culture production in pharma industry, which has similar requirements as the emerging cultured meat industry. Thus, the scaffold obtained by the method of the present invention can be sterilized by any techniques known in the art for this end, but preferably by hot steam. Consequently, this scaffold facilitates its incorporation in industrial processes requiring low cost and high-volume production, as they can be used inside large bioreactors while still using the common low-cost sterilization procedures already implemented in bioreactors or fermenters, preferably in stainless steel bioreactors or fermenters.

Thus, in a first aspect, the invention relates to a method for obtaining a sterilizable macroporous three-dimensional (3D) scaffolds which comprises a network of at least an inter-crosslinked biocompatible polymer, hereinafter the method of the invention, wherein the method comprises the following steps:
a) Preparing a dissolution of the at least a biocompatible polymer,
b) Pouring out the solution of step a) into molds and freeze it; preferably at a temperature lower than the freezing temperature of the solution,
c) Lyophilizing the freeze-scaffold obtained in step b),
d) Curing the lyophilized scaffold of step c), and
e) Sterilization of the scaffold, preferably by hot steam.

As used herein, the terms "scaffold" or "support" used interchangeably thorough the present disclosure, relate to a 3D matrix or material that allows the attachment and the proliferation of the cells, or other compounds such as additives, according to the present invention. "Attachment", "attach" or "attaches" as used herein, refers to cells that adhere directly or indirectly to the scaffold as well as to cells that adhere to other cells.

As used herein, the term "biocompatible polymer" refers to a synthetic or natural material/polymer that is, for example, non-toxic to biological systems and/or congruent with biological processes. In this respect, biocompatibility of polymer materials denotes minimal, negligible, or no risk of immuno-rejection, injury, damage and/or toxicity to living cells, tissues, organs, and/or biological systems.

In a preferred embodiment, the biocompatible polymer is a natural polymer. In an illustrative embodiment, the natural polymer, is anyone which can be able to resist a sterilization process at high pressures and temperature without degradation and/or denaturalization. Thus, the natural polymer or any derivative thereof is for example, but not limited to, polypeptides, polynucleotides, natural resins, rubbers, natural polyesters and polysaccharides. In a more preferred embodiment, the natural polymer is selected from the list consisting of: alginate, chitosan, starch, dextran, pullulan, heparin, heparin sulphate, cellulose, hemicellulose, glucomannan, agar, xanthan, guar gum, chondroitin sulphate, gelatine, chitin, a polysaccharide, a glycosaminoglycan, or any combinations thereof. In a more preferred embodiment, the natural polymer is selected from chitosan and/or alginate.

In the particular case of use gelatin as natural polymer, it is not sterilizable by hot steam and high pressure since it is partially hydrolyzed and denatured if submitted to these procedures. In the case that this natural polymer or likes, is inside the scaffold as small traces mixed with other biopolymer such as chitosan, the modification of scaffold properties is minimum still maintaining their adhesive properties.

These natural polymers can be directly used without any treatment from commercial sources or alternatively, chemical modifications can be carried out before being used to improve the properties of the scaffold of the present invention, in any of the steps of its production. As used herein, the term "derivative" is a similar compound obtained by chemically changing a part of a compound but having the same or even better properties as the original compound. Thus, the derivatives of the natural polymers of the present invention refers to chemical modifications comprising the incorporation of different groups such as amino groups, aldehydes groups, carboxylic acids groups, amides groups, ketones groups, esters groups, alkoxy groups, sulphates groups, phosphates groups or likes.

The scaffold obtained by the method of the invention may comprises the natural polymer mentioned above, having the advantage of being composed of natural biopolymers approved for food use by the competent institutions such as, for example, the U.S. Food and Drug Administration (FDA) and the European Food Safety Authority (EFSA). In this sense, the edible, sterilizable and biocompatible polymers used in the scaffold of the invention are designed so that the scaffold be edible and does not have to be extracted from the final product, i.e. in order to avoid the collection process.

As used herein, the term "edible" refers to materials, preferably food material intended for oral contact or consumption by eating.

In the present invention is highly recommended to avoid the use of natural polymer selected from the list consisting of collagen, albumin (all types and forms), caseins, hyaluronic acid, fibrin, fibronectin and elastin, among others as they are not suitable for hot steam or autoclave sterilization process.

In another preferred embodiment, the biocompatible polymer is a synthetic polymer or any variant thereof. Examples of synthetic polymers include, but not limited to, synthetic polypeptides from microorganisms or prepared by chemical synthesis techniques, bioesters, obtained from the extraction from microorganisms or prepared by chemical synthesis from their monomers such as polylactic acid, polyglycolic acid, poly(lactic-co-glycolic) acid, and polyhydroxyalkanoates, or any other synthetic polymer prepared from generally recognized natural monomers such as monosaccharides (such as glucose, fructose, manose, galactose or any derivatives or variants such as glycosamines, aminosugars,... or likes), aminoacids or any derivative, nucleotides or any derivative, fatty acids or any derivative, or likes in any combination.

In a more preferred embodiment, the molecular weight (Mw) of the biocompatible polymers, or any derivatives thereof, range from 1000 Da to 5000000 Da, preferably from 10000 to 1000000 Da, more preferably from 100000 to 500000 Da.

In a more preferred embodiment, the selected biocompatible polymer, preferably a natural polymer as mentioned above in step a), is dissolved at the required concentration, in a solvent selected from the list consisting of: aqueous solutions, organic solvents, culture media, or any combinations thereof.

In another preferred embodiment, the organic solvents are selected from the list consisting of ethanol, dioxane, acetone, acetic acid, iso-propyl alcohol, acetonitrile, dimethylsulfoxide, trifluoracetic acid or any other, as pure or as a co-solvent in aqueous solutions in any appropriate ratio.

In another preferred embodiment, the culture media refers to a solution containing nutrients to support cell survival under conditions in which the selected cells can grow. Examples of suitable culture media are selected from commercially available classical media such as Dulbecco's Modified Eagle's Medium (DMEM, MEM) or any variants thereof, including specifically defined culture mediums for each cell type used. In this sense, the skilled person knows what the specific culture media is suitable for each cell type used.

In another preferred embodiment, the concentration of the biocompatible polymer of the invention, or any combinations thereof, ranges from 0.5% to 16% (w/w), preferably between 1% to 8%, more preferably between 1.5% to 4%, or alternatively in any of the possible relative ratios when there is more than one biocompatible polymer in the final scaffold.

In another preferred embodiment, the pH of the solution comprising the solvent and the biocompatible polymer can be neutral or can be a pH which can ranges from 1 to 14, preferably from 2 to 10, to enhance the solubility of the polymer depending on the polymer used.

In order to improve the solubility of the selected biocompatible polymers, the temperature of step a) can be increased from room temperature until 180°C. In a preferred embodiment, the temperature ranges from 22°C to 70°C, more preferably from 30 to 70°C, more preferably yet, from 35°C to 65°C.

In order to provide a scaffold with improved characteristics, it can be formed and/or combined with other molecules/substances/biomolecules with high nutritional value or providing improved texture or adding flavor to the final product. In order to do that, optionally in step a) may be added to the solution any compound, substance or biomolecule able to modify or coat the surface of the scaffold to be obtained. These compounds or substances may also enhance the adhesion properties and the proliferation of the future cells which can be seeded and cultured with the scaffold. These substances are the so called cell adhesion molecules such as the inmuglobulin-superfamiliy, cadherins (E-, N-, P-,...), selectins (P-, E-, L-,...) or integrins; fibronectins, poly-L-ornithine, collagen, vitronectins, lectin, poly-ornithine, poly-L-lysine, poly-D-lysine, cyclic peptides, RGD-containing peptides (Arg-Gly-Asp), RGDS-containing peptides (Arg-Gly-Asp-Ser) or any other compound or substance recognized by an expert in the field that promotes cell adhesion to the scaffold.

Any technique, recognized by any expertise in this field, can be used to perform the surface-modification of the three dimensional porous scaffold, but immersion of the scaffolds, keeping their initial shape and dimensions or varying slightly their dimensions, in an aqueous solution or organic solvent in which the agents to be adhered are dissolved at any concentration is preferably used. If required, the temperature of solution is controlled between 22° to 180°C to improve the surface-modification process. The process time will last between 1 min to 72 h depending on the scaffold porosity, temperature, concentration of the molecule/substance to be adhered and sort of molecule/substance.

The conditions of surface-modification are controlled to obtain three-dimensional porous scaffold with a degree of coating between 0.000001 % and 100%.

To this end, in another preferred embodiment of the method of the present invention, at least a molecule/substance/biomolecule selected from an additive, bioactive molecules, and/or a cross-linker agent, or any combination thereof, can be further added to step a) in order to simplify the process of fabrication of the sterilizable 3D macroporous scaffolds disclosed herein.

Thus, in another preferred embodiment, the method of the present invention further comprises bioactive ligands which can be optionally added to step a) or after step c) of the method of the present invention. The bioactive ligands specifically interact with one or more biomolecules of cells or bind biomolecules that interact with biomolecules of the cells that are distributed within the scaffold or that proliferate within or migrate into the scaffold. Such interactions are effective to direct cell fate or induce the cells to form a tissue. These compounds or substances may also enhance the adhesion properties and the proliferation of the future cells which can be seeded and cultured with the scaffold. The identity of the bioactive ligands will depend upon the identity of the target cells, and some examples of suitable bioactive ligands include: carboxyl, amine, phenol, guanidine, thiol, indole, imidazole, hydroxyl, sulfate, norbornene, maleimide, laminin, fibrinogen, peptide sequences, adhesion molecules such as inmuglobulin-superfamiliy, cadherins (E-, N-, P-,...), selectins (P-, E-, L-,...) or integrins; fibronectins, poly-L-ornithine, collagen, vitronectins, lectin, poly-ornithine, poly-L-lysine, poly-D-lysine, cylic peptides, RGD-containing peptides, RGDS-containing peptides or any other compound or substance recognized by an expert in the field that promotes cell adhesion to the scaffold. Preferably, the bioactive molecules of the present disclosure refer to those substances that resist the sterilization process without affecting significantly their capacity to promote the cell adhesion. In a preferred embodiment, the bioactive molecule is poly-L-lysine, more preferably, poly-ε-lysine (poly-epsilon-lysine).

Poly-ε-lysine (ε-poly-L-lysine, EPL) is a naturally occurring antibacterial cationic peptide, a homopolymer of L-lysine, that is water soluble, edible, biodegradable, and non-toxic to humans and to the environment. Poly-ε-lysine is commonly used as a natural food preservative, emulsifying agent, as well as in cosmeceutical and pharmaceutical industry. In this sense, the Poly-ε-lysine is commonly used in in food applications such as boiled rice, cooked vegetables, soups, noodles, sliced fish (sushi) and meat.

This is the first time that the poly-ε-lysine is used as bioactive molecule, preferably as bioactive ligand for improving cell adhesion in *in vitro* cell cultures. As used in the present invention, the poly-ε-lysine is not only found on the surface of the scaffold of the invention, but also within it, being part of the overall 3D structure of the scaffold, thus allowing and improving cell adhesion thereon. Moreover, due to the natural antimicrobial activity of the poly-ε-lysine against yeast, fungi and bacteria, it helps to inhibit the growth of such pathogenic microorganisms in the scaffold of the invention.

In addition, poly-ε-lysine is already available in large quantities and at a low-price to the food industry, allowing the industrialized use thereof in the production of cultured meat. Thus, this property in conjunction with the surprising advantage of the scaffolds of the invention characterized in that are sterilizable, preferably by hot steam, without degradation and/or denaturalization of the biocompatible polymers selected for composition thereof, make them suitable production in large scale industrial reactors, requiring, as previously mentioned, a very low production cost.

The additive including, but not limited to molecules/substances with high nutritional value or providing improved texture or adding flavor to the final product according to the present invention, and moreover are those substances that resist the sterilization process without affecting significantly their capacity to promote the cell adhesion. The additive are selected from the list consisting of: a flavoring, a flavor enhancer, a colorant, a color enhancer, salts, acidity regulators, thickeners, emulsifiers, stabiliser, a nutritional enhancer, such as vitamins, amino acids, fiber; fructo-oligosaccharides; inulins; beta carotene; omega fatty acids; spirulinas; probiotics; prebiotics; saponins; antioxidants; essential fatty acids; minerals; and likes, or any combinations thereof. The additives selected for the scaffold of the present invention are preferably from natural origin or source. Additionally, the term additive also encompasses compounds which enhance the adhesion properties and the proliferation of the future cells which can be seeded in the scaffold.

Examples of suitable crosslinking groups are hydroxyl groups, carbonyl, groups, aldehyde groups, carboxylate group, carbonate group, carboxyl groups or derivatives, carboxamide groups, imine groups, imide groups, thiol groups and/or any other group, polyelectrolytes, or any combinations thereof.

In another preferred embodiment, the additives, adhesion molecules and/or cross-linker agents can be attached or bonded to the surface of the scaffold by physisorption or chemisorption. The process of surface-modification by the addition of additives, adhesion molecules, and/or cross-linker agents, is not limited to the outer parts of the 3D porous scaffold, but due to their intrinsic porosity and 3D, the surface-modification is extended to all the surface i) to which the modification is susceptible of happen and ii) even in the most inner part.

In another preferred embodiment, the cross-linker agents are also added to the solution of step a) of the method of the invention. The addition of cross-linker to the solution of step a) enhance the mechanical, thermal and/or stability properties of the scaffold during the production process. The crosslinking thus formed can be obtained chemically such as covalent links which might require a cross-linker agent or it can be obtained by chemical reaction between the functional groups of the polymers used, physically such as by generating non-covalent bonds, or enzymatically. The crosslinking process can be done by any of the techniques recognized by an expert in this field such as: solution, ultraviolet treatment, gamma radiation, electro-beam, thermal treatment, pressure, chemical treatment, enzymatic treatment, or likes.

The conditions of crosslinking are controlled so that the degree of crosslinking in the 3D macroporous scaffolds obtained ranges from 0.0001 % and 100%. The crosslinked scaffolds are subjected to different stages of neutralization, intensive washing (aqueous and/or other solutions), drying and freezing-lyophilisation to remove any non-desired derivative in the final scaffold.

The solution obtained in step a) of the method of the present invention, further comprising at least a molecule/substance/biomolecule selected from an additive, bioactive molecules, and/or a cross-linker agent, or any combination thereof, are washed, filtrated and/or centrifugated to eliminate any non-dissolved solid and/or compound and further to remove air bubble.

Once the solution of step a) is obtained, it is necessary to conform it to the desired shape and dimensions. Thus, in step b) of the method of the invention, the dissolution can be poured out into molds and later subject it to a freezing process, or other possibility is use other conformation techniques such as formation into shapes via extrusion directly at freeze temperatures. According to the ASTM International standards organization, extrusion is the official name given to a specific 3D printing process where material is selectively dispensed through a nozzle or orifice.

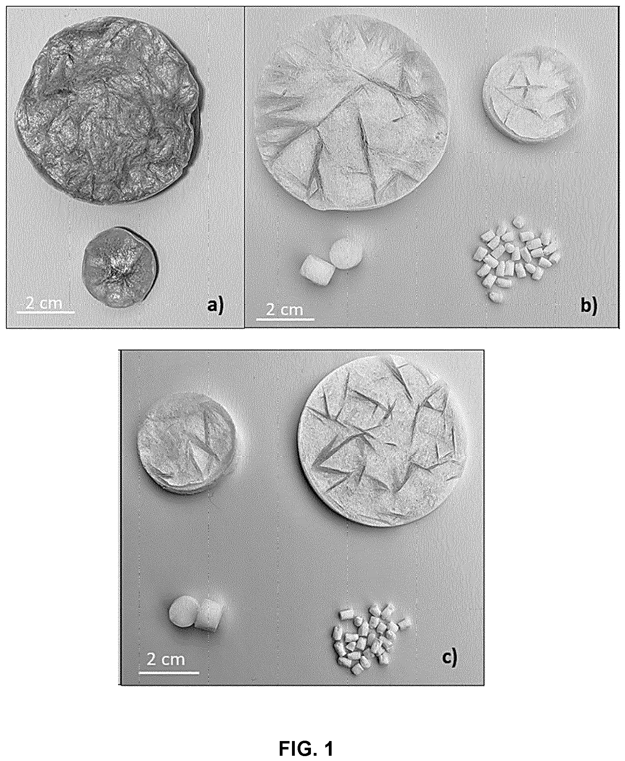

In the case of using the molds, they can be selected to confer to the scaffold the shape desired. In a preferred embodiment, the shape of the scaffold of the present invention may be regular (e.g., cylindrical, spherical, rounded, rectangular, square, pyramid, prism, cone, etc.) or irregular. In another preferred embodiment, the size of molds varies in the range of 100×100 µm to 10×10 cm (axb), preferably 1×1 mm to 5×5 mm, more preferably 1×1 cm to 3×3 cm to enhance the cellular proliferation and tissue formation. The dimensions can vary within the stated ranges in an irregular shape were a is different to b. For example, Fig. 1 illustrate variations of scaffolds having cylindrical shapes. Any of these shapes may be porous.

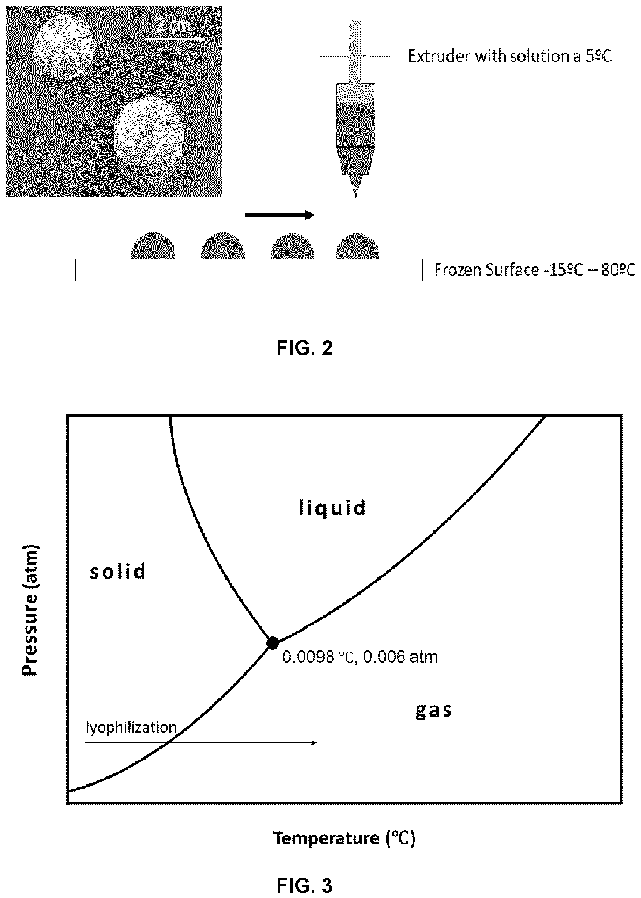

In the particular embodiment to obtain the conformation of the scaffold of the invention by extrusion, the solution of step a) is maintained at low temperature ranging from 0 to 5 °C and lately is pumped into the extruder and printed into a frozen surface from -15 to -80 °C where the drops immediately freeze as it is showed in Fig. 2. The inset of Fig. 2 shows and optical image of the scaffold obtained by the method of the invention obtained by extrusion.

As is has been mentioned above, there are a large number of variables that will determine the final properties of the scaffolds obtained by the method of the present invention, including the degree of crosslinking, the composition of the biocompatible polymer solution (i.e. whether there is a single polymer or mixtures thereof), gelation temperature and the time and temperature of the freezing process.

In this sense, the final pore size of the scaffold of the invention is influenced by the concentration of the polymer used in step a) of the method of the present invention. Furthermore, polymers with hydrogel behavior, with a high polymer concentration give rise to smaller pore sizes. In this case, the production process of the 3D porous scaffold gives longitudinal pore size range between 50 µm to 10.000 µm, preferably 100 µm to 1000 µm, more preferably 100 and 500 µm. In particular embodiments, the average longitudinal pore size is in the range of 50 to 1000 µm, 100 µm to 1000 µm, or 100 µm to 500 µm. In order to obtain macropores, a specific mold can be used in the method for the production of the scaffold of the invention, which makes it possible to obtain transverse pores. The average size of the transverse macropore ranges from 1 µm to 10000 µm, and preferably these transverse macropore are located along all the volume of the scaffold.

The arrangement of longitudinal pores, obtained through the formation of water crystals (solvent), together with the transverse macropores enables the obtainment of hydrogels with interconnected and hierarchical porous structures.

Furthermore, an important factor to be taken into account in the formation of the porous scaffold by freezing techniques, is the percentage by weight/volume of the biocompatible polymers used. In a preferred embodiment, the percentage by weight/volume of the biocompatible polymer selected ranges from 0.5% to 16%. In another preferred embodiment, the percentage by weight/volume ranges from 1% to 8 %, more preferably ranges from 1.5% to 4%, more preferably yet 1.5%, in aqueous solutions, organic solvents, cultured media, or in any combinations thereof.

Another important factor to be taken into account in the formation of the porous scaffold by freezing techniques is the temperature, preferably in step a) to obtain the dissolution and also in step b) of freezing. In this sense, when freezing is carried out at very low temperatures, preferably at a temperature which ranges from -15°C to -80°C, the solvent crystallizes faster, giving rise to the nucleation of smaller solvent crystals. Normally, the gel solution is frozen at temperatures ranging between -5 °C and -20 °C. At these temperatures, a high percentage of the solvent crystallizes; however, a portion of the gel remains in the liquid state. As the solvent is crystallised, the polymer components become concentrated in liquid microphases instead of remaining in the crystallised macrophase. Therefore, as the crystallisation of the solvent increases, the liquid microphases become more concentrated, giving rise to denser and thinner pore walls. The temperatures used in the method of the present invention, preferably which ranges from -20°C to -80°C are considered valid freezing temperatures for the formation of scaffolds of the present invention.

Once the solutions are poured to the molds, these are left to reach room temperature, if required. In another preferred embodiment, the molds comprising the solutions are frozen at a temperature which ranges from -20°C to -80°C, for a period that goes preferably from, 16 to 48 hours. These macroporous 3D scaffold, formed at a temperature lower than the freezing temperature of the dissolution, shows ease of formation compared to other macroporous structure-forming techniques makes them easily scalable to industrial production. Furthermore, the obtention of the macropores at temperatures below the freezing point of the solvent, avoids certain risks, such as the cytotoxicity of certain porogenous agents, as in the case of the formation of 3D structures using other techniques. In order to obtain larger pore sizes, a specific mold can be used, which makes it possible to obtain transverse pores ranging from microns to millimetres (along all the volume of the scaffold). The arrangement of longitudinal pores, obtained through the formation of solvent crystals, together with the transverse pores enables the obtainment of hydrogels with interconnected and hierarchical porous structures.

In a further step of the method of the invention, the lyophilization of step c) is performed for a period which preferably ranges from 16 to 96 hours, preferably from 24 to 72h, more preferably from 18 to 24h at a condenser temperature which ranges from 25°C to -100°C, preferably from 25°C to -100°C, more preferably from 20°C to -50°C, and more preferably from -15°C to -45°C, and to a pressure from 0,01 to 0,026 Pa (1 to 2.6 × 10⁻⁴ mbar), more preferably from 100 to 40 Pa (1 to 0.4 mbar), more preferably from 20 to 40 Pa (0.2 to 0.4 mbar). The parameters used in the process of lyophilization, such as time, temperature and pressure, are selected in a manner that the scaffolds loose at least 98% of the initial water. See Fig. 3 wherein the phase diagram of water indicates the triple point when lyophilization occurs. In order to help sublimation, lyophilized shelves may be heated up to 20 °C. A temperature difference of 15 °C to 20 °C between sample and condenser is recommended.

In a more preferred embodiment, after the scaffold is lyophilized it could be subjected, optionally, to different steps of neutralization if needed, such as: intensive washing (aqueous and/or other solutions) in order to remove any non-desired derivative in the final scaffold. This scaffold can be subjected, optionally, to posterior drying and freezing-lyophilisation, if needed for storage purposes.

In another preferred embodiment of the method of the present invention, at least a molecule/substance/biomolecule selected from an additive, bioactive molecules, and/or a cross-linker agent, or any combination thereof, as it has been mentioned above, can be further added to the lyophilized scaffolds obtained, in order to provide a new scaffold with the advantages mentioned herein.

In another preferred embodiment, once obtained the lyophilized scaffolds comprising the molecules/substances/biomolecules, mentioned above, the method further comprises to wash the scaffold and optionally repeating the steps b) to c), as many times as the skilled person consider necessary.

Later, the lyophilized scaffolds are subjected to a curing procedure, preferably to a thermal curing procedure, to induce the reorganization of the components of the scaffolds and to promote or enhance their mechanical, thermal and/or stability properties of the final product.

In a preferred embodiment, the thermal treatment comprises the cure of the lyophilized scaffold at a temperature which ranges from 30°C to 180°C, preferably from 50°C to 130°C more preferably from 60°C to 100°C; for a period of time which range from 1 minutes to 48 hours, preferably from 30 minutes to 8 hours. More preferably from 1 hour to 5 hours. The selected temperatures and times should prevent the decomposition of the polymeric components of the scaffold and/or the different additives used such as cell adhesion agents, crosslinkers, or likes, used in the production of the scaffolds.

In general, the lyophilized scaffolds are cured in ovens. During the curing process in the ovens, the degree of ventilation and the relative humidity are controlled by the incorporation of internal regulation systems.

The 3D macroporous scaffolds obtained by the method mentioned herein, are sterilized before the cell seeding process. The sterilization process is done by the treatment of the scaffolds by preferably, physical sterilization, chemical sterilization, or any other method known in the art and approved by the regulatory agencies in the field of food and biomedicine.

Particularly, the physical sterilization process comprises hot steam, dry heat, tyndallisation, and ionizing or non-ionizing radiation, e.g. UV radiation for surfaces, x-rays for liquids, although this procedure is very costly and safety concerns.

In a preferred embodiment for economical large-scale sterilizations of liquids and equipment the physical sterilization process is hot steam. In sterilization with hot steam, the temperature ranges from 110°C to 140°C and the times from 5 minutes to 40 minutes. The chemical sterilization process also comprises the sterilization by the application of alcohols e.g. 70% ethanol-water (pH=2), aldehydes, phenols, ethylene oxide (gas), hydrogen peroxide, 3% sodium hypochlorite, or likes.

Thus, by the method of the present invention is obtained an scaffold which can be constituted by at least a biocompatible polymer, preferably by a biocompatible natural polymer or mixtures thereof, approved for food use, having a 3D structure and a large surface area, that may serve as a support for animal cells in the production of cultured meat, even in large-scale bioreactors, and which can remain in the final product due to being composed by natural biopolymer approved for food use. Moreover, the scaffold obtained is/can be sterilized, preferably by hot steam without losing any of its properties. In this sense, the method of the invention is suitable for being integrated on cultured meat production line, contributing to the production of scaffolds that may be used later in the cultured meat production process, for example inside bioreactors.

Thus, in a second aspect the present invention relates to a scaffold, hereinafter the scaffold of the invention, obtained by the method of the present invention.

In a preferred embodiment, the scaffold is an edible and sterilizable macroporous 3D tissue engineering scaffold comprising a network of at least a biocompatible polymer, as mentioned above.

One of the main challenges of supporting large scale cultured meat growth using the scaffolding technique is the compatibility of the scaffolds with the sterilization techniques used in large scale bioreactors, as mentioned previously. Thus, the scaffolds obtained by the method of the present invention are adapted to achieve a cost-efficient process for large volume productions of food products. As it has been mentioned earlier, the sterilization process is based on a combination of steam, pressure and time. The sterilization process is performed by hot steam in an autoclave or bioreactor at temperatures which ranges from 121°C (250°F) to 134°C (273°F), and at a pressure which ranges from 0.5 to 1.05 bar, for time which range from 10 to 60 min, more preferably, the sterilization by hot steam is performed for 20 min at a temperature for at least 121°C and at a pressure of 1,05 bar to kill microorganisms and spores. The scaffold known in the art are not suitable to accomplish this process without losing its properties for tissue engineering.

On the contrary, the scaffold of the invention has the surprising advantage of being sterilizable since the biocompatible polymers selected for composition thereof are capable of resisting high water steam pressures at hot temperatures without degradation and/or denaturalization. It is important to note that the scaffold of the invention keeps intact its composition and structure after being subjected to a sterilization process, allowing proper cell adhesion and reticulation. Additionally, the sterilization process of the scaffold of the invention not only does not change the applicability of the materials due to possible degradation and/or denaturalization, but even improve the mechanical properties for supporting cell proliferation. Therefore, the scaffold of the invention could be sterilizable by any method known by the skilled in the art, including hot steam without losing its properties as scaffold for cell proliferation, facilitating its incorporation on industrial processes requiring low cost and high-volume production.

The scaffold obtained by the method of the present invention which comprises the additives and/or the cross-linkers compounds mentioned above, further may comprises living cells, preferably non-human living cells, which are homogeneously distributed though the scaffold.

Cells used for culture along the scaffold of the invention to provide the tissue with high nutritive content and/or the culture meat described herein, may include, without limitation, e.g., muscle cells, preferably smooth muscle cells; skeletal muscle cells, stromal cells, endothelial cells, satellite cells, fibroblasts cells, myoblast cells, adipose cells such as adipocyte progenitor cells, cardiomyocytes, hepatocytes etc.,. Thanks to its structure, the scaffold of the invention enables the proliferation and differentiation of various cell types such as, inter alia, myoblasts/myotubes/fibroblasts (muscle cells), adipocytes (adipose cells), yielding cultured meat suitable for human consumption with the protein-fat content desired by the end consumer as the final product.

In a further preferred embodiment, the living cell lines cultures along the scaffold could be obtained from various animal sources, such as, without limitation, mammals such as porcine, bovine, ovine, horse, dog, cat; avian; reptile; fish; amphibians; crustaceans; cephalopods or likes. This opens the doors to a huge market, since it offers a very diverse offering to end consumers.

In another preferred embodiment, the scaffold is saturated with a cell culture medium, in order to facilitates cell proliferation or survival, i.e. a cell growth medium. In some embodiments, the scaffold may be impregnated with a biomolecule or other material intended for delivery to the food, preferably meat. In a more preferred embodiment, the cell growth medium is any suitable medium containing nutrients to support cell survival under conditions in which selected cells can grow. Examples of suitable culture media are selected from any commercially available classical media such as Dulbecco's Modified Eagle's Medium (DMEM, MEM) and any variants thereof or specifically defined culture mediums for each cell type used.

The edible and sterilizable scaffold obtained by the method of the invention are appropriate for the biofabrication of tissue of nutritive content and/or cultured meat for human consumption. Although the scaffolds and methods of forming and using them described herein are primarily directed to comestible engineered meat products, such scaffold may also find application in animal (and particularly human) cell therapy and regenerative medicine, where it is desired to do cell expansion in the absence of animal-derived products. For example, described herein are edible and sterilizable macroporous 3D tissue engineering scaffolds for use in forming a cultured meat product.

Moreover, thanks to the highly macroporous structure of the scaffold obtained by the method of the invention allows the transport of nutrients, thereby facilitating cell adhesion and proliferation, and giving rise to a raw material that can be subsequently processed in food prepared for consumption. Furthermore, the scaffold distributes the cells in the complete 3D space. Until now, these scaffolds had never been envisaged for this purpose, since the large-scale cultured meat production technique is in its beginnings. The concept of these scaffold for producing cultured meat is novel and the application of the materials used to obtain the scaffold, in this form of highly porous edible and sterilizable scaffold, is also novel.

Therefore, the biocompatible polymer used in the scaffold of the present invention as mentioned above, and consequently, the scaffold of the invention comprising it, have the following advantages:
(i) it is also used as a binder, giving the growing meat better texture and compaction.
(ii) it improves the dispersal, bioavailability and absorption of nutrients in the digestive process stage;
(iii) taking into account that the biocompatible polymer could be a biopolymer with hydrogel properties, the scaffold where the cells grow can also provide a texture pleasant to the taste and touch;
(iv) it can also act as a flavour and colour booster, since it acts as a release system *per se* similar to a drug-release system (carrier);
(v) it also acts as a selective delivery system for certain nutraceuticals and/or free amino acids, thereby opening a wide range of possibilities for using these edible structures as possible carriers of vitamins, minerals, nucleic acids, or other biomolecules.

The scaffold of the invention satisfies fundamental requirements of tissue engineering, including: highly interconnected macropores promote nutrient diffusion and facilitate cell proliferation, spreading, migration and cell-cell communication also cell migration, large internal surface area produce a large capacity for the proliferation of cells; distribute cells in 3D, facilitate nutrient transport, support cell migration and proliferation and facilitate mechanical support for the newly production of tissues.

The scaffolds of the present invention, as described above, are suitable for a variety of applications, including regenerative medicine and tissue engineering. The scaffolds are particularly useful as tissue engineering. Thus, they may be used to facilitate cell proliferation and tissue formation. The macroporosity is suitable to allow cellular migration, formation of intracellular matrix, supply of nutrients, angiogenesis, and the like. The scaffold material as mentioned herein is biocompatible.

Moreover, the scaffold of the invention is compatible with various types of reactors or bioreactors, even those wherein electric currents will be applied to stimulate cell proliferation, thus, the scaffold of the invention is also electrically conductive.

Thus, in another aspect, the present invention refers to the use *in vitro* of the scaffold of the present invention in forming cultured meat products from cultured cells.

In a preferred embodiment, the cultured meat it is characterized in that the culture cells are selected from the list consisting of: mammals, avians, fish, reptils, crustaceans, cephalopods and amphibians.

In another preferred embodiment, the cultured meat it is characterized in that the cells are preferably muscle cells, and optionally, further comprises a plurality of satellite cells, stromal cells, myoblast cells, fibroblasts cells, endothelial cells, adipose cells, hepatocytes, cardiomyocytes or any combinations thereof.

In another preferred embodiment, the cultured meat it is characterized in that further comprises adipose cells, satellite cells, fibroblasts, myoblast or any combinations thereof.

In another preferred embodiment, the cultured meat it is characterized in that further comprises at least one additive selected from the group consisting of a flavoring, a flavor enhancer, a colorant, a color enhancer, a nutritional enhancer or any combinations thereof.

In another aspect, the present invention also refers to a method of forming cultured meat by the culture of living cell, preferably living muscle cells with the scaffold of the present invention, wherein the cultivation takes place in a suitable bioreactor and in the presence of a suitable nutrient culture medium.

Additionally, a particularly innovative fact is that the scaffold of the present invention may act as a carrier for a large variety of food supplements such as, for example, fiber, vitamins, amino acids, or likes, and it is suitable for sterilization processes without losing the tissue engineering scaffolding properties.

Thus, in another aspect, the present invention relates to the use of the scaffold of the present invention as a carrier.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
**Figure 1****.** Cylindrical scaffolds of different heights and diameters formed by molding. Scaffolds a), b) and c) corresponding to Example 1, 2 and 3, respectively.
**Figure 2****.** Schematic description of extrusion into a frozen surface. Inlet shows the final pellets that can be produced by this method.
**Figure 3****.** Phase diagram of water indicating the triple point; wherein the lyophilization process will occur below of this triple point.
**Figure 4****.** Scanning electron microscopy (SEM) images of porous scaffolds of Example 1 (**4a**), Example 2 (**4b**) and Example 3 (**4c**) before cell seeding. Tissue proliferation over scaffolds of Example 1 (**4d**), Example 2 (**4e**) and Example 3 (**4f**).
**Figure 5****.** Mercury (Hg) porosimetry studies before (**A**) and after (**B**) sterilization process of the scaffold disclosed in Example 1. Data of total porosity and total surface area.
**Figure 6****.** Mercury (Hg) porosimetry studies before (**A**) and after (**B**) the sterilization process of the scaffold disclosed in Example 2. Data of total porosity and total surface area.
**Figure 7****.** Mercury (Hg) porosimetry studies before (**A**) and after (**B**) sterilization process of the scaffold disclosed in Example 3. Data of total porosity and total surface area.
**Figure 8****.** FTIR (Fourier Transform-Infrared Spectroscopy). spectra of samples before (top line) and after (bottom line) sterilization procedure corresponding to scaffolds from Example 1 (**8A**), 2 (**8B**) and 3 (**8C**), respectively.
**Figure 9****.** Figure shows glucose consumption (expressed al g/L) at different assay times (expressed as days) by the mammal cells seeded in the scaffold obtained in the Examples 1 to 3 of the invention (Ex 1, Ex 2 and Ex 3, in the legend) in comparison to the initial glucose (initial, in the legend) and the cell culture controls in the absence of the scaffolds of the invention (control, in the legend).

### EXAMPLES

Following are examples of the invention by means of assays carried out by the inventors, which evidence the effectiveness of the product of the invention. The following examples serve to illustrate the invention and must not be considered to limit the scope thereof.

### Example 1. Synthesis of a scaffold, by the method of the invention, comprising chitosan as biocompatible polymer

In a typical production method of the edible and sterilizable macroporous 3D scaffold that will be equally valid for other biocompatible polymers and mixtures thereof, commercial MEM Eagle (Minimum Essential Medium Eagle) w/Earle's BSS w/Non-Essential aminoacids & L-glutamine, w/o Calcium in powder (ranging among 0-600 mg, to achieve 0-200% related to the chitosan weight, more preferably 75 mg, 10% related to the chitosan weight), was dissolved in acetic acid (0.1 M, 50 mL) and chitosan (750 mg, 1.5%) was slowly added while stirring. The mixture was stirred until homogeneous solutions were obtained (65°C, 2 hours) and the solution was poured into molds to give the desired shape and dimensions. All the samples were frozen at -20°C for 24 hours and lyophilized at -40°C during 72 hours, at a pressure of 0.263 mbar.

This is a method for manufacturing scaffolds with controlled shapes, which will be determined by the receptacle wherein the process is carried out.

The scaffolds were removed from the molds and put inside the oven at 135°C for 1 hour without ventilation (the curing starts by pre-heating the oven at 45°C, as soon as the oven reaches 135°C time starts; after 1 hour the scaffolds are left to reach room temperature inside the oven, it takes around 1h 30 minutes. At this stage, the scaffolds were washed with miliQ water (3×100 mL for 15 minutes), 0.1 M NaHCC₃ (3×100 mL for 15 minutes) and water (3×100 mL for 15 minutes).

The results show that the scaffold obtained by the method of the invention which comprises MEM powder (0%) loses its consistency when it is immersed in water. Therefore, it seems that the cross-linking temperature and time was not enough to obtain a scaffold with the desired properties regarding mechanical strength and appearance. However, the results show that the remaining scaffolds comprising different MEM concentrations (1%, 8%, 16%, 33%, 133%, 200% more preferably 8%) display good consistency and do not lose their shape. These data state that the components of the MEM are also acting as cross-linkers giving consistency to the otherwise breakable scaffolds.

Later, the scaffolds with an 8% MEM concentration were sterilized in an autoclave at 121°C, 1.05 bar, for 20 minutes and tested. The scaffolds are stable, and some degree of cross-linking has happened because the scaffolds do not swell in excess, this is the cross-linking reduces the swelling.

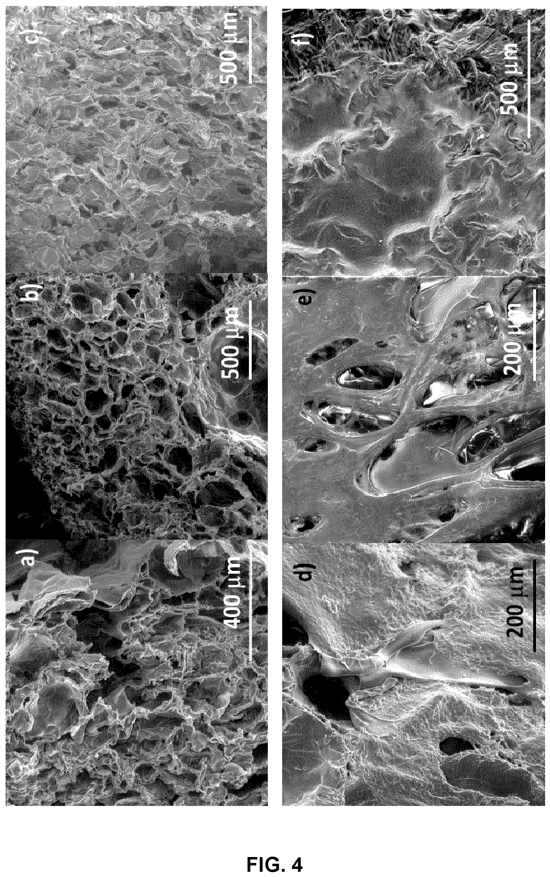

The macroporosity of these scaffolds obtained with a percentage by weight/volume of the chitosan polymer (1.5%), could be controlled and shows a high pore volume and total porosity of 90% giving rise to foam-like samples. The optimized scaffold exhibits a high volume and size of inter-connected macroporosity in the range 1-500 µm as can be observed by Digital Imaging (Fig. 1a) and SEM (Fig. 4a).

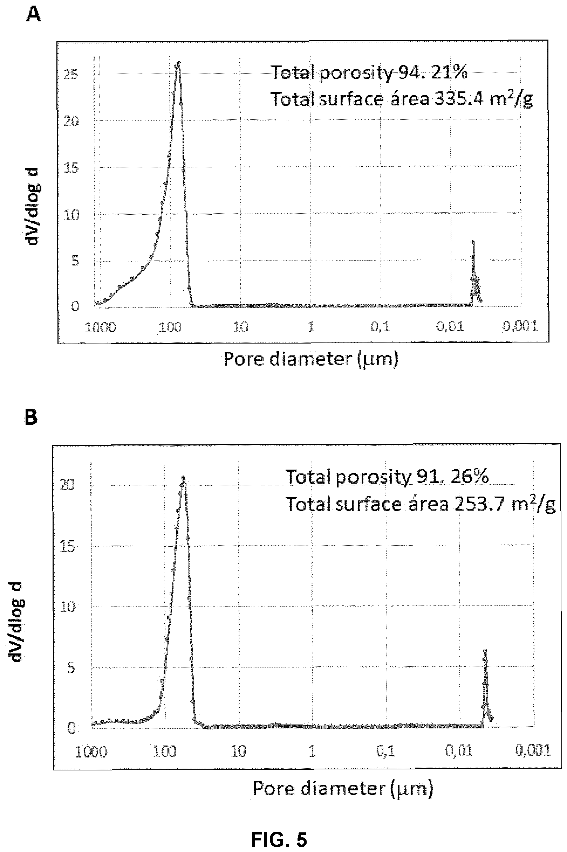

In Fig. 5 it is show the Hg (mercury) intrusion porosity analysis of the scaffold obtained in the instant example. This technique is used to analyze the total connected porosity, volume of pores, pore size distribution, and surface area of solid and powder materials. The instrument, known as a porosimeter, employs a pressurized chamber to force mercury to intrude into the voids in a porous substrate. As pressure is applied, mercury fills the larger pores first. As pressure increases, the filling proceeds to smaller and smaller pores. Both the inter-particle pores (between the individual particles) and the intra-particle pores (within the particle itself) can be characterized using this technique. The volume of mercury intruded into the sample is monitored by a capacitance change in a metal clad capillary analytical cell called a penetrometer. The sample is held in a section of the penetrometer cell, which is available in a variety of volumes to accommodate powder or intact solid pieces. Sample size is limited to dimensions of approximately 2.5 cm long by 1.5 cm wide. This powerful technique was used to analyze the change on the porous nature of the scaffold before (**Fig. 5A**) and after (**Fig. 5B**) sterilizing the pieces. As can be seen in **Fig. 5****,** results show a slight decrease of total pore volume and in the total surface area due to shrinking from the sterilization step. Still, the values after sterilization are more than highly suitable for the application showing the right pore volume range and high surface area available for cell colonization.

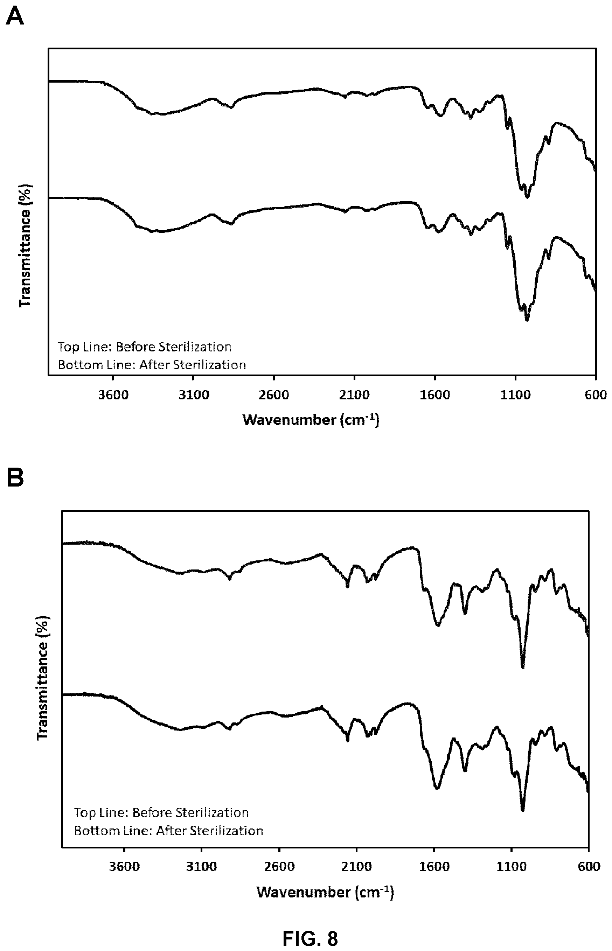

In order to prove that non-chemical modifications have occur during hot steam sterilization procedure that could compromise scaffolds suitability for cell culture, scaffolds before and after sterilization have been analyzed by FTIR (Fourier Transform-Infrared Spectroscopy). FTIR is an analytical technique used to identify organic (and in some cases inorganic) materials. This technique measures the absorption of infrared radiation by the sample material versus wavelength. The infrared absorption bands identify molecular components and structures. As it can be seen in **Fig. 8A** there are no changes on the band spectrum shape and position before and after the sterilization process proving the suitability of the chemical identity of the scaffolds.

### Example 2. Synthesis of a scaffold, by the method of the invention, comprising alginate as biocompatible polymer and coated with poly-ε-Lysine.

Alginic acid sodium salt (Sigma- Aldrich) at a concentration of 2.0 g was slowly added under vigorous stirring to water (200 mL). The mixture was stirred at 50 °C for 3 hours until a homogeneous and transparent and clear solution was obtained. The solution was left to reach room temperature.

Later, the solution was poured into molds to give the desired shape and dimensions. All the samples were frozen at -20°C for 24 hours and lyophilized at -40°C for 72 hours at a pressure of 0.263 mbar.

The lyophilized scaffolds obtained were removed from the molds and immersed in a 2% aqueous solution of calcium chloride (4 g, 200 mL) (any other aqueous solution can be used, such as, calcium sulfate, calcium carbonate, calcium gluconate, calcium citrate) in miliQ water (200 mL) for 15 minutes to provoke the crosslinking of the polymer. In this solution, the alginate scaffolds gelled immediately and washed extensively with miliQ water to remove the unbound calcium ions. The pellets were then immersed in a 0.2% poly-epsilon-lysine solution (1.0 g in 500 mL) for 16 hours, and then washed extensively with miliQ water. The scaffolds were frozen again at -20°C for 24 hours and lyophilized at -40°C for 72 hours and a pressure of 0.263 mbar.

The lyophilized scaffolds were later heated in an oven at 115°C for 1 hour without ventilation (the curing starts by pre-heating the oven at 45°C, as soon as the oven reaches 115°C time starts; after 1 hour the scaffolds are left to reach room temperature inside the oven, it takes around 1h 30 minutes), and later, stored until used.

Later, the scaffolds were sterilized in an autoclave at 121°C for 20 minutes at 1.05 bar.

The optimized scaffold exhibits a high volume and size of inter-connected macroporosity in the range 1-500 µm as can be observed by Digital Imaging (**Fig. 1b**) and SEM (**Fig. 4b**).

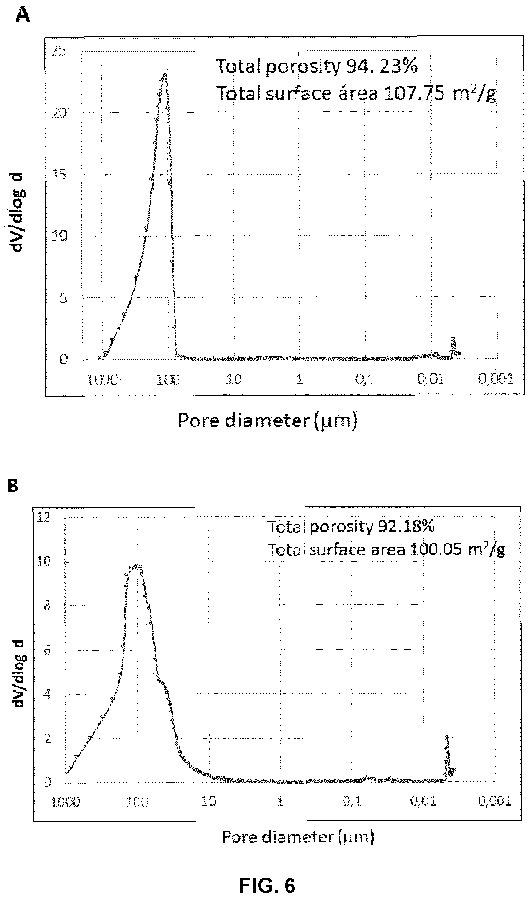

As shown in **Fig.6****,** the Hg (mercury) intrusion porosity analysis, before (**Fig. 6A**) and after (**Fig. 6B**) sterilizing the pieces, shows a slight decrease of total pore volume and in the total surface area. Still, the values after sterilization are highly suitable for the application showing the right pore volume range and high surface area available for cell colonization.

As it can be seen in **Fig. 8B** there are no changes on the band spectrum shape and position before and after the sterilization process proving the suitability of the chemical identity of the scaffolds.

### Example 3. Synthesis of a scaffold, by the method of the invention, comprising chitosan as biocompatible polymer and coated with poly-ε-Lysine

Food grade chitosan (9.0 g) was mixed with acetic acid (0.1 M, 600 mL) under gentle stirring at 40°C until chitosan was dissolved. Poly-ε-L-lysine (360 mg powder, M_{w}:1000-300000) was portion wise added to the previous solution under vigorous stirring and the mixture was warmed to 65 °C for 2 hours and 30 minutes to obtain a homogeneous and clear solution.

The previous solution was left to reach room temperature and it was poured into the molds to give the scaffolds the desired shape and size (**Fig. 1c**). The solution inside the molds was frozen at -20°C for 24 hours and lyophilized at -40°C for 72 hours and a pressure of 0.263 mbar.

The scaffolds were removed from the molds and they were jellified with ethanol absolute (2 litres) for 4 hours. The scaffolds were washed with high purity water and the scaffolds were immersed in a NaOH solution (1%) for 3 hours. The scaffolds were extensively washed with high purity water until the lixiviate reaches a neutral pH (6.5-7.5). The so formed scaffolds are frozen at -20°C for 24 hours and lyophilized (72 hours, -40°C and P = 0.263 mbar). The scaffolds were directly used without any further treatment or alternatively, it can be cured. Cured scaffolds were subjected to a heat treatment by introducing them inside an oven at 110°C for 1 hour without ventilation (the curing starts by pre-heating the oven at 45°C, as soon as the oven reaches 110°C time starts; after 1 hour the scaffolds are left to reach room temperature inside the oven, it takes around 1h 30 minutes). Later, the scaffolds were sterilized in an autoclave at 121°C for 20 minutes at 1.05 bar. The optimized scaffold exhibits a high volume and size of inter-connected macroporosity in the range 1-500 µm as can be observed by Digital Imaging (**Fig.1c**) and SEM (**Fig. 4c**).

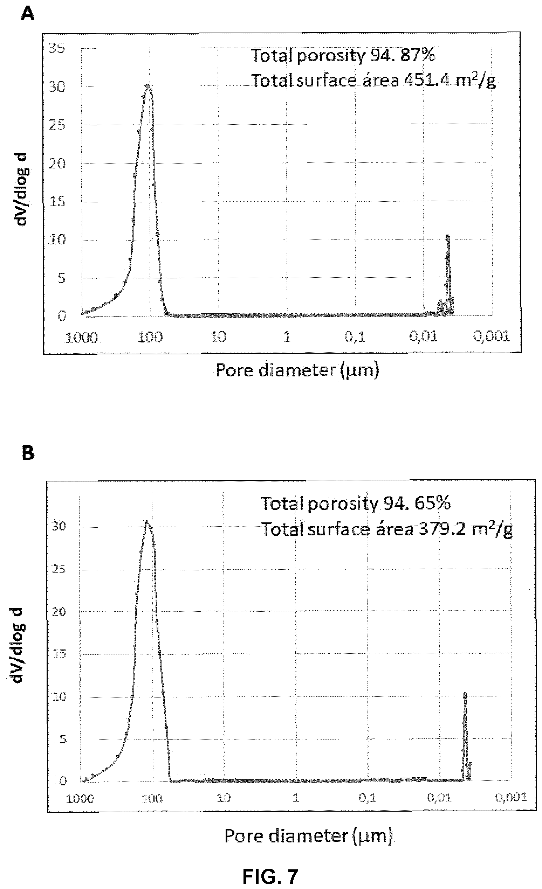

As shown in **Fig.7****,** the Hg (mercury) intrusion porosity analysis, before (**Fig. 7A**) and after (**Fig. 7B**) sterilizing the pieces, shows a slight decrease of total pore volume and in the total surface area. Still, the values after sterilization are highly suitable for the application showing the right pore volume range and high surface area available for cell colonization.

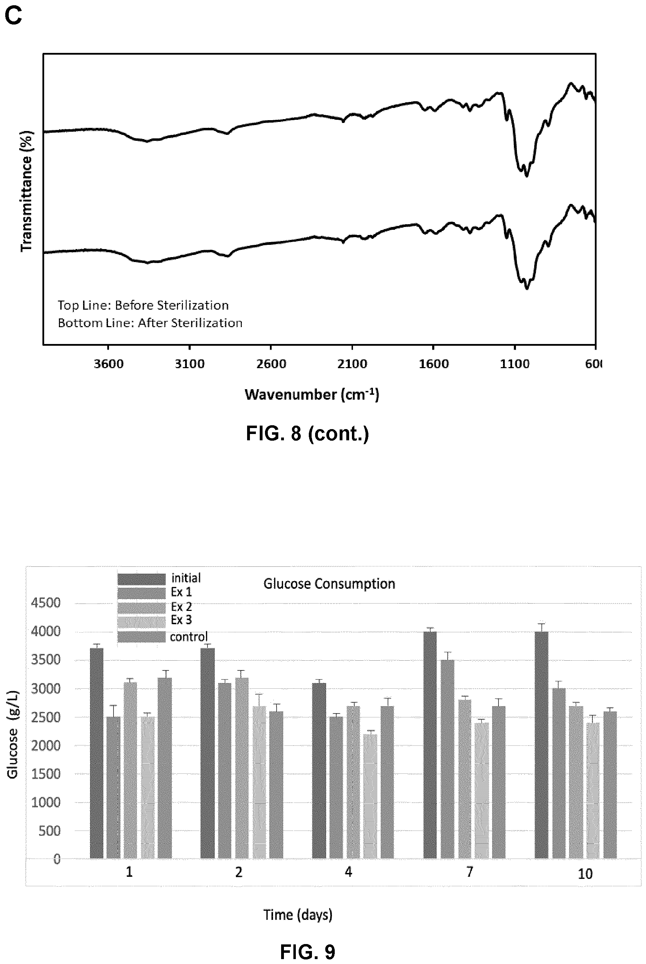

As it can be seen in **Fig. 8C** there are no changes on the band spectrum shape and position before and after the sterilization process proving the suitability of the chemical identity of the scaffolds.

### Example 4. Culture the scaffolds of Examples 1 to 3 obtained by the method of the invention with non-human cells in a bioreactor.

To verify that the chitosan based-scaffolds (Examples 1 and 3) and alginate based-scaffolds (Example 2) obtained by the method of the invention are useful for obtaining cultured meat, the inventors cultured living cells along the mentioned scaffolds of the invention in a bioreactor.

To this end, the scaffolds of the Examples 1, 2 and 3 were sterilized in an autoclave at 121°C for 20 minutes at 1.05 bar and sterilely immersed in a final volume of culture medium of 2 liters (Gibco^{™} OptiPRO^{™}). Next, a known cell density (13.000 cells/cm²) was inoculated in the mentioned scaffolds and kept under culture for long periods of time (10 days or more) in a 48-hour perfusion bioreactor. The inoculated cells were primary porcine-myoblast cells, of proprietary extraction obtained from muscle biopsies and extracted following the protocol described in JM Spinazzola et al., Bio Protoc. 2017 November 5; 7(21). The culture conditions for adequate cell proliferation require maintaining a temperature of 37 °C and a pH value of 7.1-7.4. Additionally, a continuous supply of gases is maintained, such that the concentration of dissolved oxygen ranges between 20% - 30%. After a period of 10 days, the scaffolds were removed from the bioreactor and dehydrated with ethanol in order to perform SEM (Scanning Electron Microscopy) visualization. SEM images (**Fig 4 d, e and f****)**, show the full tissue proliferation over the scaffold of Example 1, Example 2 and Example 3, respectively.

During the incubation period of 10 days, glucose consumption was measured with a Bioprofile Analyzer with a Glucose biosensor. Glucose biosensors are amperometric electrodes that have immobilized enzymes in their membranes. In the presence of oxygen and the substrate being measured, these enzyme membranes produce hydrogen peroxide (H₂O₂), which is then oxidized at a platinum anode held at constant potential. The resulting flow of electrons and current change is proportional to the sample glucose concentration. Measurements were carried out at different assay times as an indirect measurement of the proliferation and formation of tissue over the scaffold of the present invention. The data obtained are shown in **Fig. 9****.** The results show the existence of glucose consumption by the primary porcine-myoblast cells sown in the matrix of the invention over time (1-10 assay days), since a decrease in glucose concentration values is observed compared to the initial glucose levels of the culture medium (approximately 4 g/L). Positive controls were performed on each assay day.

Thus, the results show that the method of the present invention is useful to obtain a good scaffold for cell culture and for obtaining *in vitro* cultured meat.

## Claims

1. A method for obtaining a sterilizable macroporous three-dimensional (3D) tissue engineering scaffold which comprising a network of at least an inter-crosslinked biocompatible polymer, wherein the method comprises the following steps:
a) Preparing a dissolution of the at least a biocompatible polymer,
b) Pouring out the solution of step a) into molds and freeze, preferably at a temperature lower than the freezing temperature of the solution;
c) Lyophilizing the freeze-scaffold obtained in step b)
d) Curing the lyophilized scaffold of step c), and
e) Sterilization of the scaffold by hot steam.

2. The method according to 1 wherein, the biocompatible polymer is selected from natural, synthetic polymer, or any variant thereof.

3. The method according to claim 2 wherein, the natural polymer or any variant thereof is selected from the list consisting of: dextran, alginate, chitosan, starch, heparin, heparin sulfate, pullulan, cellulose, hemicellulose, glucomannan, agar, chondroitin sulfate, gelatin, chitin, polynucleotides, a polysaccharide, a glycosaminoglycan, natural polyesters, or any combinations thereof, preferably, the natural polymer or any variant thereof is selected from chitosan and/or alginate.

4. The method according to claim 2 wherein, the synthetic polymer or any variant thereof is selected from the list consisting of: polylactic acid, polyglycolic acid, poly(lactic-co-glycolic) acid, polyhydroxyalkanoates, bioesters, or any combinations thereof.

5. The method according to any of claims 1 to 4 wherein, the polymer has a molecular weight which ranges from 1000 Da to 5000000 Da, preferably from 10000 to 1000000 Da, more preferably from 100000 to 500000 Da.

6. The method according to any of claims 1 to 5 wherein, in step a) the polymer is dissolved in a solvent selected from the list consisting of: aqueous solutions, organic solvents, culture media, or any combinations thereof.

7. The method according to any of claim 1 to 6 wherein, the percentage by weight/volume of the polymer in the solution of step a) ranges from 0.5% to 16%, preferably, between 1% to 8 %, more preferably between 1.5% to 4%.

8. The method according to any of claim 1 to 7 wherein, the pH of step a) ranges from 1 to 14, preferably from 2 to 10; and the temperature of step a) ranges from 22°C to 180°C, preferably from 30°C to 70°C.

9. The method according to any claim 1 to 8 wherein the scaffold of step b) is formed by molding the dissolution via extrusion directly or by molding into molds of different shapes and sizes.

10. The method according to any of claims 1 to 9 wherein, the freezing temperature of step b) ranges from -15°C to -80°C.

11. The method according to any of claims 1 to 10 wherein, the lyophilization of step c) is performed for at least a period which ranges from 16h to 96h, preferably from 24 to 72h, more preferably from 18 to 24, at a pressure which ranges from 1 to 2.6 × 10⁻⁴ mbar, preferably from 1 to 0.4 mbar, more preferably to 0.263 mbar, and at a temperature which ranges from 25°C to -100°C, preferably from 20°C to -50°C, and more preferably from -15°C to -45°C.

12. The method according to any of claims 1 to 11, wherein, the curing process of step d) is a thermal curing process performed at a temperature which ranges from 30°C to 180°C for a period of time which ranges from 1 min to 48h.

13. The method according to any of claims 1 to 12 wherein, the sterilization of step e) can be performed optionally by a physical or chemical sterilization, preferably a physical sterilization process.

14. The method according to any of claims 1 to 13 wherein, optionally further comprises in step a) and/or in an additional step between step c) and d), the addition of at least one additive, at least an adhesion molecule, at least a cross-linker agent and/or any combinations thereof.

15. The method according to claim 14 wherein, the additive is selected from the list consisting of: flavoring, a flavor enhancer, a colorant, a color enhancer, salts, acidity regulators, thickeners, emulsifiers, stabilizer, a nutritional enhancer, probiotics, prebiotics, saponins, antioxidants, essential fatty acids, minerals, and any combinations thereof.

16. The method according to claim 14 wherein, the adhesion molecule are selected from the list consisting of: inmuglobulin-superfamiliy proteins, cadherins, selectins or integrins; fibronectins, poly-L-ornithine, collagen, vitronectins, lectin, poly-ornithine, poly-L-lysine, poly-D-lysine, cyclic peptides, RGD-containing peptides, RGDS-containing peptides or any combinations thereof, preferably the adhesion molecule is poly-ε-lysine.

17. The method according to claim 14 wherein, the cross-linker agent is selected from the list consisting of amine groups, hydroxyl groups, carbonyl groups, aldehyde groups, carboxylate group, carbonate group, carboxyl groups, carboxamide groups, imine groups, imide groups, thiol groups, inorganic ions and any combinations thereof.

18. The method according to any of claims 14 to 17 wherein further comprises washed the scaffold and optionally repeating the steps b) to c).

19. A sterilizable by hot steam and edible macroporous 3D tissue engineering scaffold which comprising a network of at least a biocompatible polymer obtained by the method according to any of claims 1 to 18.

20. Use *in vitro* of the scaffold according to claim 19 in the production of a tissue and/or a cultured meat.

21. Use according to claim 20 wherein the cultured meat comprises a plurality of adherent cells, preferably muscle cells and optionally, further comprises a plurality of satellite cells, stromal cells, fibroblasts cells, myoblast cells, endothelial cells, adipose cells, hepatocytes, cardiomyocytes, or any combinations thereof.

22. Use according to claim 21 wherein the cells belong to an animal source selected from the list consisting of: mammals preferably porcine, bovine, ovine, horse, dog, cat; avian; reptile; fish; amphibians; crustaceans, cephalopods or any combinations thereof.

23. Use *in vitro* of the scaffold according to claim 19 as carrier.
